(19) Europäisches Patentamt
European Patent Office
Office européen des brevets

(11) **EP 2 309 259 A1**

(12) **EUROPEAN PATENT APPLICATION**

(43) Date of publication:
**13.04.2011 Bulletin 2011/15**

(51) Int Cl.:
*G01N 33/38* (2006.01)    *G01N 27/04* (2006.01)
*E21B 47/00* (2006.01)    *E21B 41/00* (2006.01)

(21) Application number: **09290776.5**

(22) Date of filing: **12.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
• **Services Pétroliers Schlumberger**
  **75007 Paris (FR)**
  Designated Contracting States:
  **FR**
• **Schlumberger Technology B.V.**
  **2514 JG The Hague (NL)**
  Designated Contracting States:
  **BG CZ DE DK GR HU IE IT LT MK NO PL RO SI SK SM TR**
• **Schlumberger Holdings Limited**
  **Tortola (VG)**
  Designated Contracting States:
  **GB NL**
• **Prad Research And Development Limited**
  **Tortola (VG)**
  Designated Contracting States:
  **AT BE CH CY EE ES FI HR IS LI LU LV MC MT PT SE**

(72) Inventor: **Barlet-Gouedard, Veronique**
**92290 Chatenay Malabry (FR)**

(74) Representative: **Vandermolen, Mathieu**
**Intellectual Property Department**
**Etudes & Productions Schlumberger**
**1, rue Henri Becquerel**
**B.P. 202**
**92142 Clamart Cedex (FR)**

(54) **Methods and apparatus for monitoring cement sheath degradation related to CO2 exposure**

(57)    The present invention provides methods, apparatuses and systems for more effectively and efficiently, directly and indirectly monitoring cement degradation related to carbon dioxide exposure by measuring one or more electrical properties related to resistivity of cement behind a casing in a well.

FIG. 5

## Description

## BACKGROUND OF THE INVENTION

The present invention broadly relates to well cementing. More particularly the invention relates to a cement composition and related method of cementing for carbon dioxide environment, such as for instance a reservoir for storage of carbon dioxide gas.

In the construction of wells, cement is often used to secure and support casing inside the well and prevent fluid communication between the various underground fluid-containing layers or the production of unwanted fluids into the well. Long-term isolation and integrity of $CO_2$ injection wells clearly needs to be improved to ensure long-term environmental safety. Failure of the cement in the injection interval and above it may create preferential channels for carbon dioxide migration to the surface. This may occur on a much faster timescale than geological leakage. $CO_2$ injection well construction starts with drilling followed by well completion before starting $CO_2$ injection operations. In the framework of well completion, the cementation phase guarantees well isolation from the reservoir to the surface and isolation between geological formations. A crucial technical problem in well cementing operation or $CO_2$ sequestration is the chemical resistance of cement to $CO_2$ over time.

In addition, the environment of an underground reservoir might have high temperatures or pressures or have other hazards such as hydrogen sulfide, which might provide challenges to the material selection, well construction and/or placement process. Indeed, $CO_2$ combined with water can form carbonic acid, which could damage some conventional materials that might be used to transport, place or contain the $CO_2$, so material selection is important.

In the $CO_2$ sequestration, for example, one may want to prepare surface of the sequestration location in such a way that any leakage of $CO_2$ to the surface after placement is easily detectable and/or mitigated. It is desirable that the site be properly decommissioned after a preferably optimum amount of $CO_2$ is in place and the site monitored for sometime afterwards to ensure that the $CO_2$ placement is stable. It is desirable to ascertain the amount, location and state of the $CO_2$ during the placement process, after the $CO_2$ has been placed and for some period thereafter to ensure that the $CO_2$ stays where it has been placed and does not migrate to the surface or into drinking water aquifers. Post-decommissioning monitoring may be required by regulatory agencies.

Conventional Portland cement based systems, used during the well cementation phase, are known not to be thermodynamically unstable under $CO_2$ rich environments. This type of cement tends to degrade once exposed to such acid gases. Optimization of advanced systems allowing long-term well isolation is critical to allow safe and efficient underground activities. Indeed, any cracks or hole in the cement sheath might lead to safety problem such as "blow-out".

But $CO_2$ environment present new challenges. While facing $CO_2$ underground, being for carbon sequestration application or well cementing for isolation, leakage behind the wall of an injection well casing may provide a pathway for $CO_2$ migration into unplanned zones (other formations, adjacent reservoir zones, and other areas) potentially leading to economic loss and/or safety problems. Such $CO_2$ leakage through the annulus may occur much more rapidly than geologic leakage through the formation rock. The possibility of such leaks raises considerable concern about the long-term wellbore isolation and the durability of hydrated cement that is used to isolate the annulus across the producing and injection intervals in $CO_2$-related wells.

The long-term $CO_2$ sequestration is a more recent concept but also requires long-term wellbore integrity. Indeed, the major risk associated by the public with $CO_2$ injection is a wellbore failure, which may result in escape of $CO_2$ that will migrate upwards; most cement being not thermodynamically stable under carbon dioxide environment.

The present invention provides methods, apparatuses and systems for more effectively and efficiently, directly and indirectly monitoring cement degradation related to carbon dioxide exposure by measuring one or more electrical properties related to resistivity of cement behind a casing in a well.

## SUMMARY OF THE INVENTION

The present invention also relates to the detection of any change in the cement sheath integrity in any well application. This might be, indeed, for oil-well, water storage, carbon sequestration or any gas containment.

The present invention relates to a method for measuring the integrity of the cement sheath involving resistivity measurement in situ. Such measurement would allow detecting any changes in the cement integrity especially when it reacts with $CO_2$.

An object of the present invention is to provide methods, apparatuses and systems for transporting, measuring, sequestering carbon, as well as site preparation and design, decommissioning a carbon sequestration site and providing post-decommissioning monitoring of the sequestration site, while eliminating or minimizing the impact of the problems and limitations described.

Other objects, features and advantages of the present invention will become apparent to those of skill in art by reference to the figures, the description that follows and the claims.

## BRIEF DESCRIPTION OF THE DRAWINGS

**FIG. 1** is a flowchart for a generalized $CO_2$ sequestration process.

FIG. 2 is a flowchart for a $CO_2$ sequestration process, including an embodiment of the instant invention.

FIG. 3 is a simplified depiction of a well

FIG. 4 is a graph depicting evolution of Portland cement's resistivity with time in $CO_2$ fluids at 90°C temperature and 280 bars pressure.

FIG. 5 is a graph plotting the imaginary part of conductivity of a Portland cement before and after $CO_2$ exposure, both to wet supercritical $CO_2$ and to $CO_2$ saturated water.

**DETAILED DESCRIPTION OF THE DRAWINGS**

In the following detailed description of a preferred embodiment and other embodiments of the invention, reference is made to the accompanying drawings. It is to be understood that those of skill in the art will readily see other embodiments and changes may be made without departing from the scope of the invention.

Chemical reactions between cement and carbon dioxide occur as the following:

- $CO_2 + H_2O \leftrightarrow {}_2CO_3 \leftrightarrow H^+ + HCO_3^- \leftrightarrow 2H^+ + CO_3^{2-}$ which corresponds to the dissociation of carbon dioxide in water formation
- the following reactions correspond to the reaction between cement and carbon dioxide as dissolution and carbonation

  o $Ca(OH)_2(s) + 2H^+ + CO_3^{2-} \rightarrow CaCO_3(s) + 2H_2O$ (where $2H_2O$ is free water)
  • $C_{3\cdot4}\text{-}S_2\text{-}H_8(s) + 2H^+ + CO_3^{2-} \rightarrow CaCO_3(s) + SiO_xOH_x(s)$ (where $SiO_xOH_x(s)$ is silica gel)
  o $Ca(OH)_2(s) + H^+ + HCO_3^- \rightarrow CaCO_3(s) + 2H_2O$
  o $C_{3\cdot4}\text{-}S_2\text{-}H_8(s) + H^+ + HCO_3^- \rightarrow CaCO_3(s) + SiO_xOH_x(s)$

- Calcite dissolution under acidic condition

  o $CO_2 + H_2O + CaCO_3(s) \leftrightarrow Ca^{2+} + 2HCO^{3-}$
  o $2H^+ + CaCO_3(s) \rightarrow CO_2 + Ca^{2+} + H_2O$

Cement alteration may be characterized in laboratory by a complex series of concentric carbonation fronts after $CO_2$ attack. This chemical alteration is a very effective process with a significant pH decrease of interstitial fluid triggering possibly casing corrosion. An initial sealing by carbonation is followed by a dissolution stage, which starts earlier in $CO_2$-saturated water than in wet supercritical $CO_2$. After six months, the degradation is very high and shows a "spalling effect" (cement material starts to peel out). The evaluation of this alteration may be performed with different measurements on set cement samples as mechanical properties evolution but also as pH, porosity, permeability and resistivity before and after $CO_2$ exposure.

The cement alteration firstly controlled by the

$CO_2$ diffusion through the cement matrix is followed by a mechanical alteration resulting from the carbonation and the dissolution of calcite, creating micro-cracks in the cement. The micro cracks could accelerate $CO_2$ leakage up to the surface as a preferential pathway for $CO_2$.

As the carbon sequestration is probably the conditions where the cement is likely to be in contact with long period and at high concentration, this will be used as a preferred embodiment in the following disclosure; however, the method according to the present invention is applicable to any cemented well in contact with $CO_2$.

The concept of $CO_2$ generation and sequestration are nowadays pretty well understood. Large-scale $CO_2$ sources, for example, ethanol plants, cement factories, steel factories, refineries, electricity generation plants, coal and biomass operations generate $CO_2$ and usually vent it as atmospheric $CO_2$. Some large scale $CO_2$ sources may transport the $CO_2$ to factories where the $CO_2$ can be used for industrial processes or for food and drink products. Some $CO_2$ is sequestered through natural processes such as from trees "inhaling" $CO_2$ and "exhaling" $O_2$ (oxygen). But $CO_2$ may be taken to a $CO_2$ capture facility, where the $CO_2$ is processed and then geologically sequestered. In geologic sequestration, the $CO_2$ is injected down one or more wells and into an appropriate formation, such as a coal seam where the $CO_2$ can displace methane to a production well by which the methane can be produced. Another appropriate formation might be a suitable depleted oil and/or gas reservoir. Another appropriate formation might be a suitable reservoir with trapped oil that the $CO_2$ may be used to displace to a production well by which the oil may be produced. Another appropriate formation might be a saline reservoir with a suitable trapping structure.

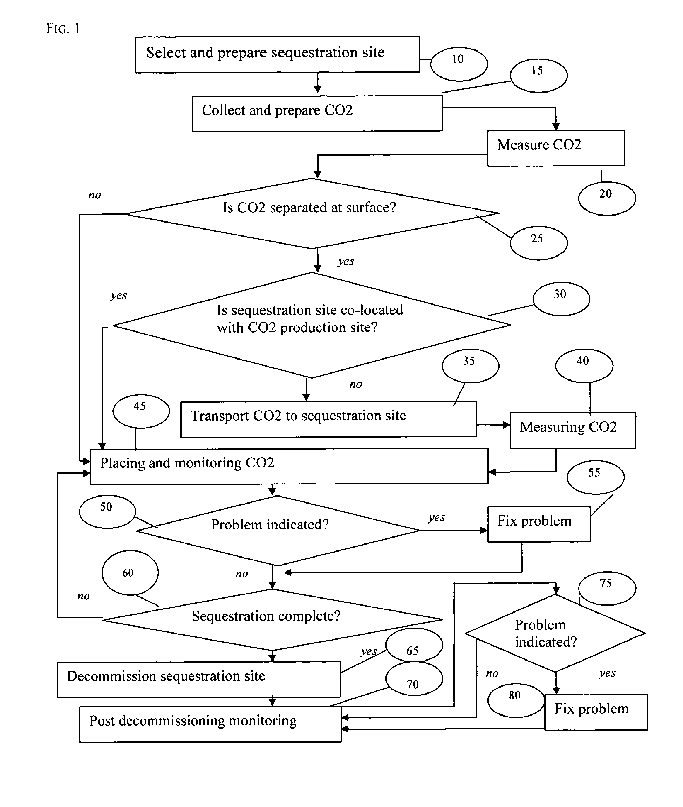

FIG. 1 is a depiction of an overview of a $CO_2$ sequestration process, which may be used with or without embodiments of the instant invention. One selects and prepares **12** a sequestration site. This may be a lengthy and involved process involving many steps and considerations. If the $CO_2$ is being produced with hydrocarbons, it can be separated underground and be sequestered without reaching the surface. If the $CO_2$ is being collected at the surface, a determination would be made as to whether there was a place to sequester the $CO_2$ onsite or whether the $CO_2$ might have to be transported to another site. If transportation is necessary, the possible sites available would be considered along with their relative advantages and disadvantages.

Referring again to **FIG. 1,** $CO_2$ is collected and prepared **15** for storage. How the $CO_2$ is collected and prepared depends in part on the source of the $CO_2$. The source of the $CO_2$ may be a power plant, refinery, factory or other industrial site or the source of the $CO_2$ may be from hydrocarbon production. There are countless sources of $CO_2$, including every human being and animal on the planet and most automobiles, but carbon sequestration is currently most practical where the $CO_2$ is produced in large amounts and can be easily collected. The source

of the $CO_2$ helps to determine whether the $CO_2$ requires processing, such as removal of moisture or other contaminants before transportation and/or sequestration. The $CO_2$ may be measured **20** as and/or after the $CO_2$ is collected and/or processed.

Referring again to **FIG. 1,** the process may be different, depending on whether the $CO_2$ is separated at the surface **25** and whether the sequestration is co-located with the $CO_2$ production site **30.** If the $CO_2$ is not separated at the surface but rather underground as it is produced, for example with hydrocarbons, it might be possible to have the $CO_2$ placed and monitored **45** underground. In the more typical case of collecting and separating the $CO_2$ at the surface, then a determination is made as to whether **30** the selected sequestration site is co-located with $CO_2$ production site. If the $CO_2$ is co-located with the sequestration site the $CO_2$ may still need to be transported a short distance and measured as part of being placed and monitored **45,** but if the sequestration site is not co-located with the $CO_2$ production site substantial transportation system **35,** such as through a pipeline system, may be necessary. Measurements **40** may have to be taken at several points to ascertain that there is no $CO_2$ leakage occurring or to determine a location of any $CO_2$ leakage or simply to determine the amount of $CO_2$ at various points in the process.

Continuing to refer to **FIG. 1,** at the sequestration site, the $CO_2$ is placed generally through injection wells and monitored **45.** Buffering may be provided to store $CO_2$ in case the sequestration site has to be temporarily shut down. As monitored sequestration progresses, if problems are indicated **50,** they are evaluated and ameliorated or if possible, completely fixed **55.** Monitoring may be used to determine whether the sequestered $CO_2$ is stable or whether the sequestered $CO_2$ is becoming permanently affixed in the storage formation. Sequestration continues until complete **60.** Completion might occur for example, if the source of the $CO_2$ permanently shuts down or if the storage formation cannot accept any more $CO_2$. The sequestration site would then be properly decommissioned **65** and post-decommissioning monitoring **70** might begin. If the post-decommissioning monitoring detects **75** a problem, the problem situation may have to be improved or fixed **80.**

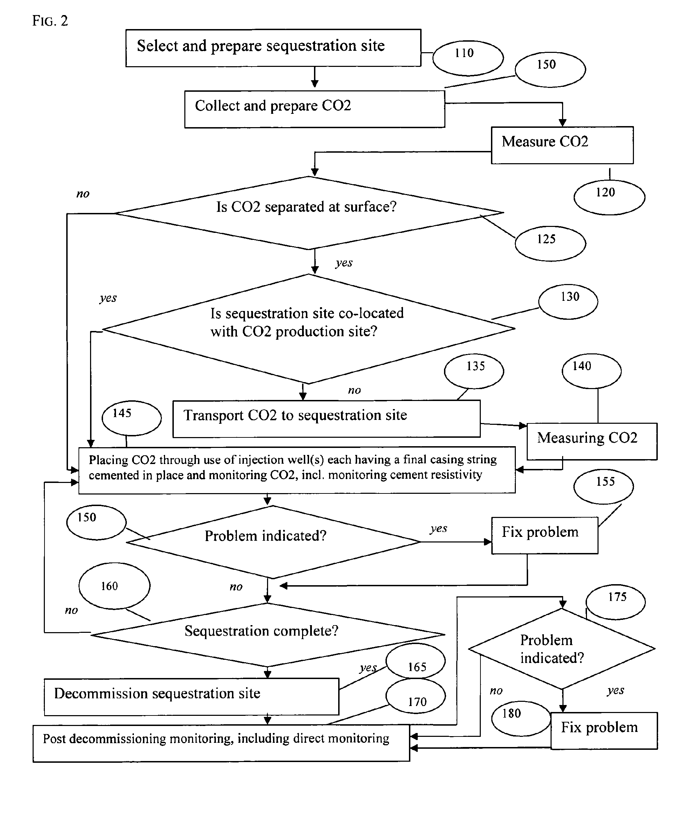

**FIG. 2** is a flowchart for a $CO_2$ sequestration process, including an embodiment of the instant invention. As with the general process presented in **FIG. 1,** one selects and prepares **110** a sequestration site. This may be a lengthy and involved process involving many steps and considerations. If the $CO_2$ is being produced with hydrocarbons, the $CO_2$ might be separated underground and be sequestered without reaching the surface. If the $CO_2$ is being collected at the surface, a determination would be made as to whether there was a place to sequester the $CO_2$ onsite or whether the $CO_2$ might have to be transported to another site. If transportation is necessary, the possible sites available would be considered along with their relative advantages and disadvantages.

Referring again to **FIG. 2,** $CO_2$ is collected and prepared **115** for storage. How the $CO_2$ is collected and prepared depends in part on the source of the $CO_2$. The source of the $CO_2$ may be a power plant, refinery, factory or other industrial site or the source of the $CO_2$ may be from hydrocarbon production. The source of the $CO_2$ helps to determine whether the $CO_2$ requires processing, such as removal of moisture or other contaminants before transportation and/or sequestration. The $CO_2$ may be measured **120** as and/or after the $CO_2$ is collected and/or processed.

Referring again to **FIG. 2,** the process may be different, depending on whether the $CO_2$ is separated at the surface **125** and whether the sequestration is co-located with the $CO_2$ production site **130.** If the $CO_2$ is not separated at the surface but rather underground as it is produced, for example with hydrocarbons, it might be possible to have the $CO_2$ placed and monitored **145** underground. In the more typical case of collecting and separating the $CO_2$ at the surface, then a determination is made as to whether **130** the selected sequestration site is co-located with $CO_2$ production site. If the $CO_2$ is co-located with the sequestration site the $CO_2$ may still need to be transported a short distance and measured as part of being placed and monitored **145,** but if the sequestration site is not co-located with the $CO_2$ production site substantial transportation system **135,** such as through a pipeline system, may be necessary. Measurements **140** may have to be taken at several points to ascertain that there is no $CO_2$ leakage occurring or to determine a location of any $CO_2$ leakage or simply to determine the amount of $CO_2$ at various points in the process.

Continuing to refer to **FIG. 2,** at the sequestration site, the $CO_2$ is placed through one or more injection wells and monitored **145.** The injection wells have a final string of casing which has been cemented into place. In accordance with an embodiment of the present invention, the monitoring process includes measurement of one or more electrical properties related to resistivity of the cement used for the final string of casing. The measurement of the one or more electrical properties related to resistivity may be performed one different ways, some of which are further discussed herein. Buffering may be provided to store $CO_2$ in case the sequestration site has to be temporarily shut down. As monitored sequestration progresses, if problems are indicated **150,** they are evaluated and ameliorated or if possible, completely fixed **155.** Monitoring may be used to determine whether the sequestered $CO_2$ is stable or whether the sequestered $CO_2$ is becoming permanently affixed in the storage formation. Sequestration continues until complete **160.** Completion might occur for example, if the source of the $CO_2$ permanently shuts down or if the storage formation cannot accept any more $CO_2$. The sequestration site would then be properly decommissioned **65** and post-decommissioning monitoring **170** might begin. If the post-decommissioning monitoring detects **175** a problem, the problem situation may have to be improved or fixed **180.**

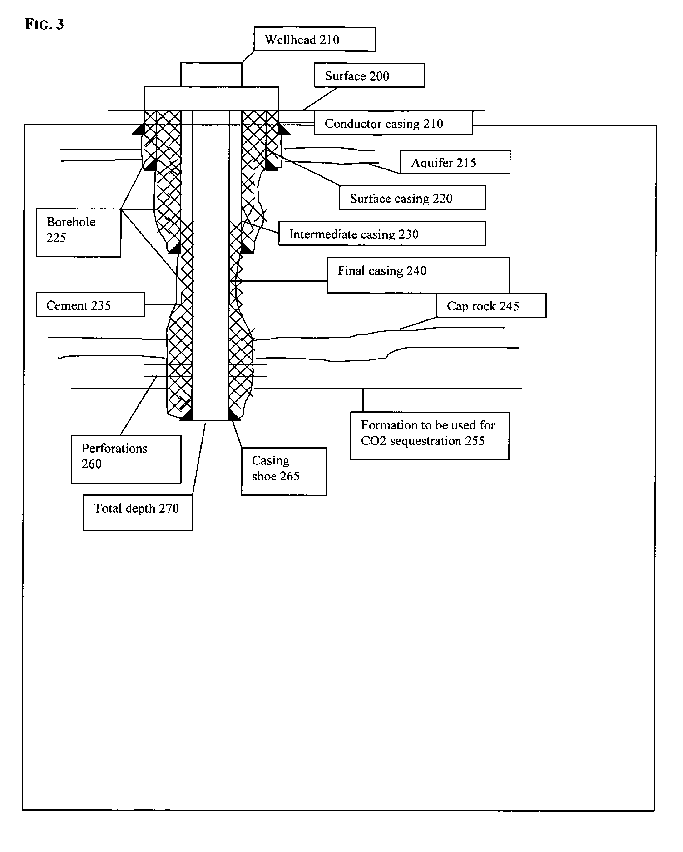

In an embodiment of the present invention, the injection well is drilled using conventional methods. **FIG. 3** is a simplified depiction of a well (not to scale). The well is begun by having conductor casing **210** placed in the ground. Sometimes the well is drilled to the depth selected for conductor casing **210** and sometimes conductor casing **210** is pounded into the ground. Conductor casing **210** is placed to provide support for the injection well and is often placed at shallow depths, such as 18 to 35 meters or so. Using a bit smaller than the conductor casing **210,** drilling of the well continues, usually through aquifers **215** until a predetermined depth for surface casing **220** is reached. The hole that is created while the well is being drilled is called the borehole **225.** Surface casing **220** is set and cemented **235** into place, with the cement being pumped down the inside of the casing, through a casing shoe **265** on the bottom of the surface casing and then filling in the space ("annulus") between the borehole **225** and outside surface of the surface casing **220** wall. Generally, for the surface casing, sufficient cement is used to bring the cement all the way back to the surface **200.** The surface casing is designed to provide support for further drilling of the injection well and to protect for example the aquifers **215**, preferably all aquifers **215** the injection well would traverse.

Continuing to refer to **FIG. 3,** using a bit smaller than the inside diameter of the surface casing, drilling of the well continues. Sometimes setting and cementing one or more intermediate casings **230** may be necessary, depending on the formations traversed by the injection well, their anticipated pressures and other hazards such as swelling clays and the total depth **270** of the injection well. If an intermediate casing is set, the bits used to drill below the intermediate casing are smaller than the internal diameter of the intermediate casing. In this case, the well is drilled through a cap rock **245,** which forms a barrier to $CO_2$ migration, and through a formation to be used for $CO_2$ sequestration **255** to total depth **270.**

At total depth **270,** after evaluating the formations traversed by the injection well, a final casing **240** is set and cemented into place. In **FIG. 3,** the total depth **270** is deeper than the formation to be used for sequestration **255,** shown along with its cap rock **245.** After the cement **235** is put into place and sets properly, the final casing **240** is perforated, so that perforations **260** provide access for $CO_2$ injection from the inside of the final casing of the injection well through the cement and into the formation to be used for $CO_2$ sequestration **255.** Although a full string of final casing extending from the total depth of the well to the surface is depicted in **FIG. 3,** sometimes liners that extend from the total depth only up to a hanger set near the bottom of in the last string of intermediate casing may be used, as is well known in the art. Similarly, although one particular well configuration is depicted in **FIG. 3,** many other configurations are possible as is well known in the art, including but not limited to different types of directional wells, horizontal wells, multiple completions of various kinds.

As mentioned earlier, the present invention is applicable to all cementing operations downhole; a type of cement commonly used for cementing for example casings in place is Portland cement. As Portland cement is exposed to $CO_2$, the Portland cement generally becomes more porous and may allow the $CO_2$ to leak through the Portland cement and migrate to the surface. Another situation might be a carbonation identified below a cap rock; this would usually be an indication of the formation of a micro-annulus allowing $CO_2$ circulation along the well. In the context of the present invention, by cap rock, it is to be understood an impermeable layer which is above the reservoir where $CO_2$ will be stored. In the petroleum industry, caprock is generally referred to as any not permeable formation that may trap oil, gas or water, preventing it from migrating to the surface. This caprock or trap can create a reservoir of oil, gas or water beneath it and is a primary target for the petroleum industry.

Measurements of the resistivity of the cement sheath in situ, according to the present invention, would allow detecting changes in cement integrity when it reacts with carbon dioxide. In fact, the variation of the real part of cement's conductivity (which is the inverse of resistivity) is indicating any changes in the cement sheath during it carbonation and/or dissolution process with $CO_2$. The cement resistivity measurement would allow an "early warning" of $CO_2$ leakage through the monitoring of the degradation of the cement behind casing during the life of a well but also after its abandonment.

Different methods for measuring the resistivity of the cement sheath below or above the cap rock, might be used by the one skilled in the art; examples are cased holed measurements (through the casing) or resistivity in cement involving instrumented casing or sensors outside casing.

The electrical resistivity of sound Portland cement is generally around 10-20 Ohm.m. The evolution of cement resistivity after its exposure to $CO_2$ fluids (e.g. wet supercritical $CO_2$ fluid or $CO_2$ dissolved in water or in brine) has been studied and basically it evolves and increases significantly with the carbonation rate depending to the $CO_2$ exposure duration. The inverse of cement resistivity being cement conductivity, it is thus possible to measure the ability of cement to conduct electricity. Mathematically speaking, conductivity calculations may include the square root of a negative number, which means conductivity may have a real part and an imaginary part. Analysis of the imaginary part of cement's conductivity shows that frequency dependency decreases in direct proportion along with cement's carbonation. This change is correlated to porosity evolution of cement when it reacts with carbon dioxide present or stored in the reservoir. In other words, one can tell whether cement is becoming damaged by the $CO_2$, if one can measure changes in the cements electrical properties, like resistivity and/or conductivity.

Porosity of a Portland cement after $CO_2$-attack

at several durations (0 hour, 44 hours, 1, 3, 6 weeks and 6 months) in wet supercritical $CO_2$ and in $CO_2$ dissolved in water fluids have been determined through mercury intrusion porosimetry, at 90°C under 280 bars. Results are reproducible, and the trend observed is similar to the one obtained by back-scattered electron imaging. In both fluids, it has been shown that, in a first period, which lasts about 3 weeks, the initial porosity (35%) of Portland cement decreases up to 9% in $CO_2$ dissolved in water and 17% in wet supercritical $CO_2$, before starting to increase after 6 weeks (14 % and 20% respectively) . This is due to carbonation, followed by a dissolution process. The threshold diameter, which is correlated to the permeability, tends to show the same behaviour: an opening of the pore entrance. These porosity results confirm the same trend as that observed with other physical parameters such as weight, density, pH, compressive strength, chemical analysis, and/or microstructural characterisations, i.e. that Portland cement continuously evolves during the carbonation process. It is noteworthy that pH of interstitial water strongly decreases from 13 initially to 6 during the carbonation process. It has to be noted that Portland cement, being widely used in the oilfield industry, is predominantly cited in the present application but the present invention is also applicable to $CO_2$ resistant cement such as Evercrete™ available from Schlumberger.

The pH of water in equilibrium with Portland cement cores initially equals to 13. After $CO_2$ attack, core samples are stored in water and the pH is measured at equilibrium. After each $CO_2$ attack, the new equilibrium pH is around 7 up to 3 weeks and decreases again up to 6 after six weeks and 3 months of experiment. Such $CO_2$ attack-related decrease of pH has already been reported in the literature (Van Gerven et al., 2004a; 2004b). This decrease results from the reaction between $CO_2$ and calcium from the calcium silicate phases or portlandite coming from Portland cement hydration.

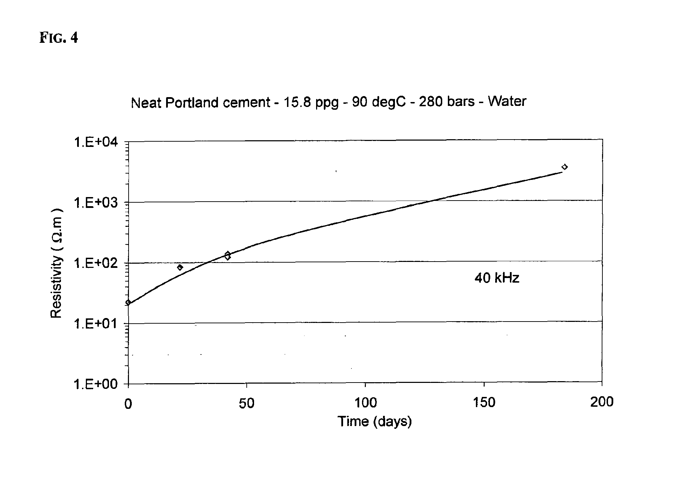

Fig. 4 is a graph depicting evolution of Portland cement's resistivity with time in $CO_2$ fluids at 90°C temperature and 280 bars pressure. Fig. 4 indicates the resistivity of the cement exposed to $CO_2$ increasing over time, from about 20 ohm-meters at the beginning of the experiment to about 100 ohm-meters after about 3 weeks and to about 1 000 ohm-meters after more than 150 days, in the experiment. In a Portland cement, as for any porous medium, the porosity variation and the composition of the interstitial water inside this porosity will affect its resistivity. The measurements of electrical resistivity of the cements included the use of the Impedance Phase/Gain Analyser SOLARTRON™ 1260 which have been used in the frequency band from 0.1 Hz to 0.3 MHz. As for conventional method of electrical impedance measurement, a 2-electrodes scheme was implemented to provide a homogeneous electric field in cement samples to be tested. Before measurements, each device had been calibrated to the open and closed electrical circuits, the resistance and capacitance of the measuring cell had been automatically compensated from the measured values. The electrical resistivity had been obtained from electrical impedance measurements Re[Zp] and Im[Zp] and samples'geometrical factor. A Portland cement with 35% porosity has a low resistivity around 20 Ohms.m [measured at a frequency of 40 kHz]. As the porosity decreases with a factor of 4 after 3 weeks (21 days) in $CO_2$ dissolved in water, a high variation of resistivity occurs (Fig. 4): the resistivity increases from 20 Ohms.m to 100 Ohms.m after 3 weeks and to 1000 Ohms.m after 6 months (168 days). In other words, as cement started to react with $CO_2$, the ability of the cement to conduct electricity is also damaged, with resistivity increasing and conductivity decreasing. Thus a cement resistivity measurement or conductivity measurement provides a method of "early warning" for the monitoring of $CO_2$ leaks through the monitoring of the degradation of the cement behind casing (sometimes called a cement sheath) during the life of the well but also after its abandonment. By monitoring the resistivity of cement sheath during the life of the well and even after its abandonment, cement degradation under carbon dioxide may be tracked over with time, yielding a good indication of the level of the carbonation.

A method to calculate the resistivity of the cement according to the present invention is described below:

Impedance spectroscopy (IS) is a relatively new and powerful method of characterizing many of the electrical properties of materials and their interfaces using electronically conducting electrodes. The impedance is a complex number that can be written as follows:

$$Z(\Box) = Z' + j\ Z''$$

with Z' being the real part of Z($\Box$), Re(Z) and Z" the imaginary part of Z($\Box$), Im(Z). The modulus of the impedance equals:

$$\left| Z(\omega) \right| = \left[ (Z')^2 + (Z'')^2 \right]^{1/2}$$

The conductivity spectrum can be obtained from impedance spectrum by using the following equation:

$$\sigma(\omega) = \frac{l}{A_e \times \left| Z(\omega) \right|}$$

with $\Box$ being the conductivity (S/m), $\Box$the frequency (Hz), |Z($\omega$)| the absolute value of impedance, I the spacing between electrodes (meters) and $A_e$ the electrode cross-sectional area (in meters squared, $m^2$).

As conductivity is the inverse of resistivity, at each frequency $\square$, the resistivity in Ohm-meters is calculated from the conductivity spectrum. Moreover, the complex dielectric constant $\square$ is also derived from impedance spectrum measurements, with the following equation:

$$\varepsilon = \frac{1}{i\omega Z(\omega)\varepsilon_0 A_e l}$$

with $\square_0$ being the permittivity of free space (= $8,884.10^{-12}$ F/m).

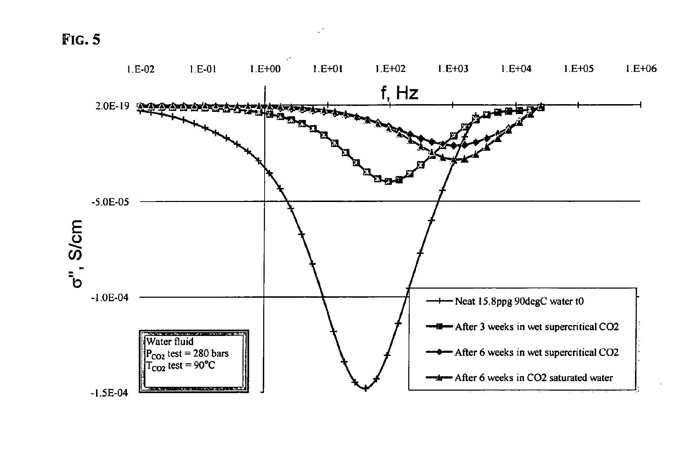

**Fig. 5** is a graph plotting the imaginary part of conductivity of a Portland cement before and after $CO_2$ exposure, both to wet supercritical $CO_2$ and to $CO_2$ saturated water. **Fig. 5** indicates a decrease in the imaginary part of the conductivity with increased times of $CO_2$ exposure. Moreover, the frequency dependence of the "imaginaire" part of conductivity (which is the inverse of resistivity) disappears after carbon dioxide exposure: this signal can be also used as an indicator of the cement reaction with carbon dioxide.

Different methods are available to measure electrical properties of the cement placed behind or inside the casing, below or above the caprock, in accordance with embodiments of the invention. The measurement may be of resistivity, conductivity or any other property of the cement from which resistivity or conductivity may be derived. The measurements may be made once or repeatedly over time, as a time lapse monitoring. Measurements may include, for example, cased-holed measurements (from the inside of the casing) which might be performed using cased-hole logging tools, or the measurements could be made using instrumented casing or sensors placed outside the casing. With the logging tools, a log may be produced and might evidence, for example cement resistivity of the cement sheath as a function of depth. While casing can interfere with resistivity measurements, resistivity of the cement sheath could be determined, behind the casing.

Sensors on the outside of the casing could be placed at one or at more than one different depths for measurements or placed in one or more configurations around the casing for measurements of the cement sheath in different directions. Useful set up can be found in US Patent No. 5,642,051, particularly Fig 2A of that patent and the discussion of Fig. 2A therein. The sensors on the outside of the casing could have electrodes as described in US Patent No. 5,642,051 and instead of measuring properties of the fluids in a reservoir, could measure resistivity of the casing sheath. Signals could be sent from the sensors on the outside of the casing to the surface. When the measurements indicate cement degradation, methods to repair the cement or other proactive measures may be used to reduce or eliminate the chance of $CO_2$ migration towards the surface.

Although the foregoing is provided for purposes of illustrating, explaining and describing certain embodiments of the invention in particular detail, modifications and adaptations to the described methods, systems and other embodiments will be apparent to those skilled in the art and may be made without departing from the scope or spirit of the invention.

**Claims**

1. A method to evaluate the cement sheath integrity related to resistivity evolution of cement when exposed to $CO_2$.

2. The method of claim 1 wherein one of the one or more measurements of the electrical property of the cement sheath related to resistivity is a resistivity measurement.

3. The method of claim 1 wherein one of the one or more measurements of the electrical property of the cement sheath related to resistivity is electrical conductivity measurement.

4. The method of claim 1 wherein one of the one or more measurements of the electrical property of the cement sheath related to resistivity is an induction measurement.

5. The method of claim 1 wherein the measurement of the electrical property of the cement sheath related to resistivity is taken inside or behind the casing.

6. The method of claim 1 wherein the measurement of the electrical property of the cement sheath related to resistivity is taken with one or more sensors installed on the outside of the casing.

7. The method of claims 5 and/or 6 wherein the casing cemented into place is a final casing in the well.

8. A method of selective cement remedial treatment comprising the steps of:

    (i) Monitoring the integrity of a cement sheath;
    (ii) Identifying a defect to said cement sheath;
    (iii) Pumping remedial compounds at the location where the defect was identified.

9. The method of claim 1 wherein the well is an injection well for injecting $CO_2$ into a formation.

10. The method of claim 1 wherein the well is a monitoring well for monitoring the $CO_2$ sequestration process.

**11.** An apparatus for measuring an electrical property related to resistivity of a cement sheath behind a casing in a well comprising:

> a. A sensor having at least one electrode installed on the outside of the casing so that the electrode is in contact with the cement, such that the electrode measures an electrical property of the cement related to resistivity of the cement; and
> b. Communication means from the sensor to the surface.

**12.** Apparatus according to claim 11, further comprising a plurality of sensors having at least one electrode which are disposed on a support adapted to maintain a given spacing between the sensors and to isolate the sensors electrodes from one another.

**13.** Apparatus according to claim 11, wherein said support is formed by a rigid metal tube with an electrically insulating coating.

**14.** Apparatus according to claim 11, wherein said support is formed by an elongate member of non-rigid, electrically insulating material.

**15.** A method of sequestering $CO_2$ comprising:

> a. collecting carbon dioxide and preparing the carbon dioxide for sequestration;
> b. selecting a storage site;
> c. obtaining permits for using the storage site for sequestration;
> d. preparing the storage site, including drilling at least one injection well having a casing cemented in place so that a cement sheath is in place between the final casing and a borehole wall of the well;
> e. transporting the carbon dioxide to the storage site, measuring the carbon dioxide as desirable;
> f. placing and monitoring $CO_2$, until sequestration is complete, including checking for problems and addressing the problems as needed, wherein the monitoring of $CO_2$ includes taking one or more measurements of an electrical property of the cement sheath related to resistivity of the cement sheath;
> g. decommission sequestration site; and
> h. monitoring the decommissioned site for leaks.

Fig. 1

Fig. 2

**FIG. 3**

Wellhead 210

Surface 200

Conductor casing 210

Aquifer 215

Surface casing 220

Borehole 225

Intermediate casing 230

Final casing 240

Cap rock 245

Cement 235

Formation to be used for CO2 sequestration 255

Perforations 260

Casing shoe 265

Total depth 270

FIG. 4

Neat Portland cement - 15.8 ppg - 90 degC - 280 bars - Water

40 kHz

FIG. 5

Europäisches
Patentamt

European
Patent Office

Office européen
des brevets

## EUROPEAN SEARCH REPORT

Application Number

EP 09 29 0776

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RIMMELE G ET AL: "Heterogeneous porosity distribution in Portland cement exposed to CO2-rich fluids" CEMENT AND CONCRETE RESEARCH, PERGAMON PRESS, ELMSFORD, NY, US, vol. 38, no. 8-9, 1 August 2008 (2008-08-01), pages 1038-1048, XP022734453 ISSN: 0008-8846 DOI: 10.1016/J.CEMCONRES.2008.03.022 * abstract; figure 8 * * page 1038, paragraph Introduction * * page 1047, paragraph Discussion * ----- | 1-7,9,10 | INV. G01N33/38 G01N27/04 E21B47/00 E21B41/00 |
| Y | FERREIRA R M ET AL: "NDT measurements for the prediction of 28-day compressive strength" NDT & E INTERNATIONAL, BUTTERWORTH-HEINEMANN, OXFORD, GB, vol. 43, no. 2, 25 September 2009 (2009-09-25), pages 55-61, XP026833876 ISSN: 0963-8695 * abstract; figures 1,3 * * page 56, left-hand column * * page 58, paragraph 4.2.1 * ----- | 1-7,9,10 | |
| A | US 2003/016009 A1 (LI ZONGJIN [HK] ET AL) 23 January 2003 (2003-01-23) * abstract; figure 5 * * paragraphs [0003], [0028], [0034] * ----- | 1-7,9-14 | TECHNICAL FIELDS SEARCHED (IPC) G01N E21B |

-/--

The present search report has been drawn up for all claims

| Place of search | Date of completion of the search | Examiner |
|---|---|---|
| Munich | 25 October 2010 | Hanisch, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 09 29 0776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BARLET-GOUEDARD V ET AL: "Mitigation strategies for the risk of CO2 migration through wellbores" PROCEEDINGS SPE/IADC DRILLING CONFERENCE 2006, MIAMI BEACH, FL, US, vol. IADC/SPE 98924, 21 February 2006 (2006-02-21), pages 1-17, XP008093505 * abstract; figures 7,8,10-12,16-18 * * page 1, right-hand column * * page 4, paragraph Results * | 1-7,9, 10,15 | |
| X | US 6 675 895 B1 (SHEHAB GAMAL [US] ET AL) 13 January 2004 (2004-01-13) * abstract; claims 1,3,7 * * column 2, lines 1-14 * * column 4, line 38 - column 5, line 48 * * column 7, lines 11-16 * | 8 | |
| X,D | US 5 642 051 A (BABOUR KAMAL [FR] ET AL) 24 June 1997 (1997-06-24) * abstract; claims 12-15 * * column 4, line 8 - column 5, line 45; figures 2A, 2B, 3 * | 11-14 | TECHNICAL FIELDS SEARCHED (IPC) |
| Y | MAY D H ET AL: "Identifying and correcting for high-resistivity cement effects for cased-hole resistivity-log analysis" PROCEEDINGS - SPE ANNUAL TECHNICAL CONFERENCE AND EXHIBITION, ATCE 2006, 24-27 SEPTEMBER 2006, SAN ANTONIN, TX, US; SOCIETY OF PETROLEUM ENGINEERS, US, vol. 1, 2006, pages 66-76, XP009140299 * abstract; figures 1, 2, 10 * * page 68, left-hand column, paragraph 3 * | 15 | |

-/--

The present search report has been drawn up for all claims

| Place of search | Date of completion of the search | Examiner |
|---|---|---|
| Munich | 25 October 2010 | Hanisch, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 09 29 0776 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WELLS A W ET AL: "The use of tracers to assess leakage from the sequestration of CO2 in a depleted oil reservoir, New Mexico, USA" APPLIED GEOCHEMISTRY, ELSEVIER LTD., GB, vol. 22, no. 5, 6 May 2007 (2007-05-06), pages 996-1016, XP022063723 ISSN: 0883-2927 DOI: DOI:10.1016/J.APGEOCHEM.2007.01.002 * abstract * * page 997, left-hand column * * page 1015, left-hand column * ----- | 15 | |
| | | | TECHNICAL FIELDS SEARCHED (IPC) |

The present search report has been drawn up for all claims

| Place of search | Date of completion of the search | Examiner |
|---|---|---|
| Munich | 25 October 2010 | Hanisch, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 09 29 0776

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 09 29 0776

The Search Division considers that the present European patent application does not comply with the
requirements of unity of invention and relates to several inventions or groups of inventions, namely:

1. claims: 1-7, 9, 10

   Method for evaluating the cement sheath integrity related to
   resistivity evolution of cement when exposed to CO2
   
   ---

2. claim: 8

   Method for selective cement sheath remedial treatment
   
   ---

3. claims: 11-14

   Apparatus for measuring an electrical property related to
   resistivity of a cement sheath behind a casing in a well
   
   ---

4. claim: 15

   Method of sequestering CO2
   
   ---

EP 2 309 259 A1

## ANNEX TO THE EUROPEAN SEARCH REPORT
## ON EUROPEAN PATENT APPLICATION NO.

EP 09 29 0776

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2010

| Patent document cited in search report | | Publication date | Patent family member(s) | | Publication date |
|---|---|---|---|---|---|
| US 2003016009 | A1 | 23-01-2003 | CN | 1399130 A | 26-02-2003 |
| US 6675895 | B1 | 13-01-2004 | NONE | | |
| US 5642051 | A | 24-06-1997 | AU | 689502 B2 | 02-04-1998 |
| | | | AU | 7884594 A | 25-05-1995 |
| | | | BR | 9404474 A | 11-07-1995 |
| | | | CA | 2135961 A1 | 18-05-1995 |
| | | | DE | 69421673 D1 | 23-12-1999 |
| | | | DE | 69421673 T2 | 15-06-2000 |
| | | | DK | 656547 T3 | 22-05-2000 |
| | | | DZ | 1824 A1 | 17-02-2002 |
| | | | EP | 0656547 A1 | 07-06-1995 |
| | | | FR | 2712627 A1 | 24-05-1995 |
| | | | GB | 2284062 A | 24-05-1995 |
| | | | NO | 944378 A | 18-05-1995 |
| | | | OA | 10112 A | 18-12-1996 |
| | | | ZA | 9409096 A | 21-07-1995 |

## REFERENCES CITED IN THE DESCRIPTION

**Patent documents cited in the description**

- US 5642051 A